# EUROPEAN PATENT SPECIFICATION

(11) **EP 1 648 521 B1**
(45) Date of publication and mention of the grant of the patent: **21.10.2009**
(21) Application number: 04778979.7
(22) Date of filing: 22.07.2004
(51) Int. Cl.: A61K 48/00, C12N 5/10, A61N 1/362, C07K 14/705

(54) **COMPOSITIONS AND METHODS FOR TREATING CARDIAC DYSFUNCTION**
ZUSAMMENSETZUNGEN UND METHODEN FÜR DIE BEHANDLUNG VON HERZERKRANKUNGEN
COMPOSITIONS ET PROCEDES POUR LE TRAITEMENT DE DYSFONCTIONNEMENT CARDIAQUE

(30) Priority: 24.07.2003 US 626159
(43) Date of publication of application: 26.04.2006
(73) Proprietor: MEDTRONIC, INC., Minneapolis, MN 55432-5604 (US)
(72) Inventor: SHARMA, Vinrod, Blaine, Minnesota 55449 (US)
(74) Representative: Hughes, Andrea Michelle
(86) International application number: PCT/US2004/023717
(87) International publication number: WO 2005/009477

(56) References cited:
- WO-A-02/087419
- US-A1- 2002 155 101
- DONAHUE J K ET AL: "FOCAL MODIFICATION OF ELECTRICAL CONDUCTION IN THE HEART BY VIRAL GENE TRANSFER" NATURE MEDICINE, vol. 6, no. 12, December 2000 (2000-12), pages 1395-1398, XP001182423 ISSN: 1078-8956
- PRIORI S G ET AL: "From catheters to vectors: the dawn of molecular electrophysiology." NATURE MEDICINE, vol. 6, no. 12, December 2000 (2000-12), pages 1316-1318, XP002303156 ISSN: 1078-8956
- MURATA MITSUSHIGE ET AL: "Calcium channel suppression by gene transfer of Kir/Gem into heart cells: Creation of a genetic calcium channel blocker." CIRCULATION, vol. 106, no. 19 Supplement, 5 November 2002 (2002-11-05), pages II-8, XP009038949 ISSN: 0009-7322
- DONAHUE J K: "Ion channel regulation: from arrhythmias to genes to channels (to cures)" DEVELOPMENTS IN CARDIOVASCULAR MEDICINE, vol. 242, 2002, pages 159-165, XP001184087
- MIAKE JUNICHIRO ET AL: "Biological pacemaker created by gene transfer." NATURE, vol. 419, no. 6903, 12 September 2002 (2002-09-12), pages 132-133, XP002303157 ISSN: 0028-0836
- SILVA JONATHAN ET AL: "Mechanism of pacemaking in IK1-downregulated myocytes." CIRCULATION RESEARCH, vol. 92, no. 3, 21 February 2003 (2003-02-21), pages 261-263, XP002303158 ISSN: 0009-7330 cited in the application
- NUSS H B ET AL: "Overexpression of a human potassium channel suppresses cardiac hyperexcitability in rabbit ventricular myocytes" JOURNAL OF CLINICAL INVESTIGATION, vol. 103, no. 6, March 1999 (1999-03), pages 889-896, XP002288643 ISSN: 0021-9738
- MARBÁN EDUARDO: "Cardiac channelopathies." NATURE, vol. 415, no. 6868, 10 January 2002 (2002-01-10), pages 213-218, XP002303159 ISSN: 0028-0836
- SCHRAM GERNOT ET AL: "Differential distribution of cardiac ion channel expression as a basis for regional specialization in electrical function." CIRCULATION RESEARCH, vol. 90, no. 9, 17 May 2002 (2002-05-17), pages 939-950, XP002303160 ISSN: 1524-4571
- QU JIHONG ET AL: "Expression and function of a biological pacemaker in canine heart." CIRCULATION. 4 MAR 2003, vol. 107, no. 8, 4 March 2003 (2003-03-04), pages 1106-1109, XP002303161 ISSN: 1524-4539
- HOSHIJIMA MASAHIKO ET AL: "Chronic suppression of heart-failure progression by a pseudophosphorylated mutant of phospholamban via in vivo cardiac rAAV gene delivery." NATURE MEDICINE, vol. 8, no. 8, August 2002 (2002-08), pages 864-871, XP002303162 ISSN: 1078-8956

## Description

The present invention relates to compositions and apparatuses for providing curative therapy for cardiac dysfunction, and more particularly to biological systems and methods relating to implementing curative therapeutic agents and systems for arrhythmias and cardiac pacing dysfunction.

In a normal, healthy heart, cardiac contraction is initiated by the spontaneous excitation of the sinoatrial ("SA") node, located at the junction of the right atrium and superior vena cava ("SVC"). The electrical impulse generated by the SA node travels to the atrioventricular ("AV") node where it is transmitted to the bundle of His and Purkinje network, which branches in many directions to facilitate simultaneous contraction of the left and right ventricles.

A depolarizing wave front gives rise to contractions among the myocytes of the atria and ventricles. During normal contraction, the left and right atria simultaneously contract followed by the simultaneous contraction of the left and right ventricles to eject oxygenated blood from the left ventricle to the aorta.

In certain disease states, the heart's ability to properly pace the atria and ventricles is compromised. For example, during atrial fibrillation, which is believed to be the result of multiple reentrant wave fronts in the atria, atrial activity is rapid, arrhythmic, and disorganized. Also, during atrial fibrillation the atrial chambers contract at rates as fast as 300-600 times per minute and the irregular electrical activity is transmitted randomly to the ventricles via the AV node resulting in rapid and irregular ventricular rhythm. Currently, one of the therapeutic strategies for such dysfunction is radio frequency ("RF") ablation of the AV node with a concurrent implantation of an implantable pacemaker to provide ventricular pacing. While this ablate and pace strategy helps to improve the lives of many patients, it is a radical and irreversible procedure that renders the AV node dysfunctional for the remainder of the patient's life.

Alternatively, atrial fibrillation has been treated with pharmaceuticals. For example, beta blockers have been used to control the ventricular rate by modulating AV node conduction without attempting to terminate atrial fibrillation. Another therapeutic strategy is to use pharmaceuticals, such as procainimide, quinidine or disopyramide, to restore sinus rhythm and thus terminate atrial fibrillation. However, the effectiveness of such drugs varies dramatically among patients and hence cannot be employed as a universal therapeutic strategy.

Developments in genetic engineering have resulted in methods for genetically modifying cardiac cells to influence their intrinsic pacing rate and treat cardiac dysfunction. For example, U.S. Patent No. 6,214,620 describes a method for suppressing excitability of ventricular cells by overexpressing (e.g. K⁺ channels) or underexpressing certain ion channels (e.g. Na⁺ and Ca²⁺ channels). PCT Publication No. WO 02/087419 describes methods and systems for modulating electrical behavior of cardiac cells by genetic modification of inwardly rectifying K⁺ channels (I_{Kl}) in quiescent ventricular cells. PCT Publication No. WO 02/098286 describes methods for regulating pacemaker function of cardiac cells with HCN molecules (HCN 1, 2, or 4 isoforms of the pacemaker current Iᵣ). U.S. Published Application No. 20020155101 A1 discloses methods of preventing or treating cardiac arrhythmias by administering a polynucleotide to cardiac cells that modulates an electrical property of the heart. Methods for genetic modification of cardiac cells of the conduction system are disclosed in commonly assigned, co-pending U.S. Patent Applications Serial Number 10/424,080 and 10/423,595, both entitled "Genetic Modification of Targeted Regions of the Cardiac Conduction System", (Docket Nos. P-11205 and P- 11273).

A need remains, however, to implement a system of genetic ablation in cooperation with either genetic modification therapy (biopacing) or pacing using an implantable medical device (IMD) or both to insure successful curative therapy for cardiac dysfunction.

The present invention provides a kit as defined in claim 1 for use in treating cardiac dysfunction, In one embodiment, the bio-ablation procedure is used in combination with a biological pacemaker ("bio-pacemaker") that is capable of responding to physiological signals as well as facilitating and restoring synchronous contractions of the ventricles to thus mimic the function of a healthy heart or in combination with an IMD. The bio-ablation procedure is performed and the bio-pacemaker is generated through the genetic modification of myocardial cells. In one aspect of the invention, a bio-ablation composition is administered to myocardial cells. The composition includes one or more coding sequences that decrease the expression of proteins in those myocardial cells responsible for cellular excitability so that impulses cannot be conducted to the ventricles during rapid atrial activity such as during atrial fibrillation. The composition may be delivered to cells of the AV node.

According to another aspect, a bio-pacemaker composition is administered to myocardial cells to generate a bio-pacemaker to replace the loss of impulses that would normally be conducted through the AV node or other bio-ablated cells. The bio-pacemaker composition includes two or more coding sequences that encode one or more molecules in myocardial cells that increase the pacemaking rate of the cells. Generally, a bio-pacemaker composition is used to genetically modify myocardial cells to increase their pacing rate to a level resembling the intrinsic pacing rate of the SA nodal cells in a normal heart. The bio-pacemaker composition generally includes polynucleotides, molecules, or modified cells containing one or more coding sequences of the invention and is delivered to the cells, via catheters, direct injection, or equivalent delivering means.

In one embodiment, the bio-ablation composition of the invention is used in combination with an implantable pacemaker. The implantable pacemaker is used to provide pacing in the heart at a location downstream from the AV node. In another embodiment, the bio-ablation method and bio-pacemaker composition are used in combination with the implantable pacemaker. The implantable pacemaker is programmed to work in conjunction with the genetically engineered bio-pacemaker to prevent cardiac dysfunction or to monitor the pacemaking action of the genetically engineered bio-pacemaker. Further, the implantable pacemaker operates to pace the heart when the pacemaking action of the bio-pacemaker is not as expected. '

Preferred embodiments will now be described by way of example only, with reference to the drawings.
Figure 1 is a diagram of a human heart.
Figure 2 is a schematic diagram of a right side of a heart, similar to Figure 1, in which a guiding catheter is positioned for delivery of the genetic construct of the invention.
Figures 3A and 3B are schematics illustrating how an embodiment of the invention operates.
Figure 3C illustrates how upregulation of ND^{a+} -Ca²⁺ exchanger (NaCaX) helps to improve the autonomic response of the bio-pacemaker obtained predominantly by suppressing I_{Kl}.
Figure 4A illustrates the use of a small implantable backup pacemaker working in cooperation with the bio-pacemaker of the invention.
Figure 4B is a logic flow diagram depicting the operational logic of the invention.
Figure 5 is a schematic of the tripartite rAAV producer plasmid, pTP-D6deltaNot.

The present invention relates to biological treatment of cardiac dysfunction by genetic ablation of the AV node. The biological ablation is combined with a pacing treatment. Pacing treatments useful with the invention include the creation of a biological pacemaker and/or implantation of an implantable pacemaker.

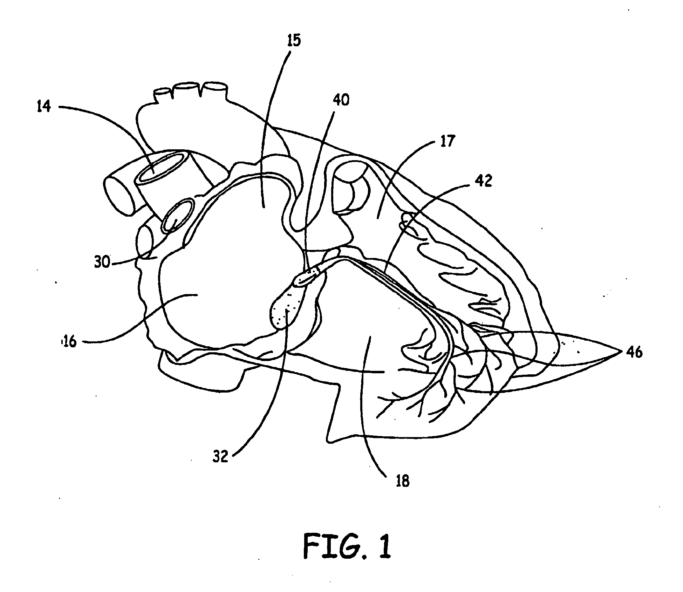

Figure 1 is a schematic diagram of a right side of a heart having an anterior-lateral wall peeled back to expose a portion of a heart's intrinsic conduction system and chambers of a right atrium 16 and a right ventricle ("RV") 18. Pertinent elements of the heart's intrinsic conduction system, illustrated, in Figure 1, include a SA node 30, an AV node 32, a bundle of His 40, a right bundle branch 42, and Purkinje fibers 46. SA node 30 is shown at a junction between a superior vena cava 14 and right atrium ("RA") 16. An electrical impulse initiated at SA node 30 travels rapidly through RA 16 and a left atrium (not shown) to AV node 32. At AV node 32, the impulse slows to create a delay before passing on through a bundle of His 40, which branches, in an interventricular septum 17, into a right bundle branch 42 and a left bundle branch (not shown) and then, apically, into Purkinje fibers 46. Following the delay, the impulse travels rapidly throughout RV 18 and a left ventricle (not shown). Flow of the electrical impulse described herein creates an orderly sequence of atrial and ventricular contraction to efficiently pump blood through the heart. When a portion of the heart's intrinsic conduction system becomes dysfunctional, efficient pumping is compromised.

Typically, a patient, with a dysfunctional SA node who does not suffer from atrial fibrillation and has an intact AV node 32 may have a pacemaker system implanted with a lead placed in an atrial appendage 15. The lead stimulates RA 16 downstream of nonfunctional SA node 30 and the stimulating pulse travels to AV node 32, bundle of His 40, and Purkinje fibers 46 and results in a cardiac contraction. However, if a patient has a non-functional, e.g., ablated AV node 32, pacing in atrial appendage 15 will not be effective, and the pacing lead will need to be placed downstream of the AV node, for example, in the RV apex or LV epicardium.

Of particular interest is pacing from the bundle of His 40. Pacing at the bundle of His 40 provides opportunities of utilizing the normal conduction system of the heart to carry out ventricular depolarizations. In other words, stimulation provided at the bundle of His will propagate rapidly to the entire heart via the right bundle 42, the left bundle (not shown), and the Purkinje fibers. This provides a more physiological ventricular contraction, which is synchronized and efficient, unlike pacing from the apex of the RV where the electrical energy propagates at a slower rate because myocardial tissue is a slow conductor compared to the rapidly conducting Purkinje network. Thus, bundle of His, upper bundle branches or Purkinje network are the most proficient regions for generation of a biological pacemaker.

To transform cardiac system cells that have no or little intrinsic pacemaking activity into a biological pacemaker, it is important to understand the electrophysiology of cardiac cells with a particular emphasis on the SA node cells. Like cells of other excitable tissue, cardiac cells allow a controlled flow of ions across their membranes. This ion movement across the cell membrane results in changes in transmembrane potential, which is a trigger for cell contraction. Each cell type in the heart, for example, atrial, ventricular, etc. has its own characteristic variation in membrane potential. For example, ventricular cells have a resting potential of ∼-85mV. In response to an incoming depolarization wave front, these cells fire an action potential with a peak value of∼20mV and then begin to repolarize, which takes ∼250-350 ms to complete. Once repolarized, ventricular cells remain quiescent until excited by another incoming depolarizing wavefront, i.e., they have no intrinsic automacity. In contrast, SA nodal cells do not have a stable resting potential and instead begin to spontaneously depolarize when their membrane potential reaches - 50mV. Cells, such as SA nodal cells, that do not have a stable resting transmembrane potential, but instead increase spontaneously to the threshold value, causing regenerative, repetitive depolarization, are said to have automacity.

The cells in the SA node are unique because not only do they have automacity, but also their firing rate is the highest among all cardiac cells that demonstrate automacity (e.g., AV node and Purkinje cells). Depolarization from the SA node cells spreads across the atria. Since atrial muscle cells are not connected intimately with ventricular muscle cells, conduction does not spread directly to the ventricle. Instead, atrial depolarization enters the AV node, and after a brief delay, is passed on to the ventricles via the bundle of His and Purkinje network, initiating synchronous excitation of the ventricles.

The SA node's unique cells include a combination of ion channels that endow it with its automacity. A review of the features of cardiac electrical function and description of the current understanding of the ionic and molecular basis, thereof, can be found in Schram et al., "Differential Distribution of Cardiac Ion Channel Expression as a Basis for Regional Specialization in Electrical Function," Circ. Res., Vol. 90, pages 939-950 (2002).

Briefly, some of the unique features of the SA node cells include the absence of Na⁺ channels (I_{Na}) and inwardly rectifying K⁺ (I_{Kl}) channels. In the absence of sodium current, the upstroke of SA node action potential is primarily mediated by L-type Ca²⁺ channels (I_{CaL}). SA node cells do not have a stable resting potential because of the lack of the I_{Kl} and begin to depolarize immediately after the repolarization phase is complete. The maximum diastolic potential for SA node cells is approximately -50 mV compared to -78 mV and -85 mV for atrial and ventricular cells, respectively. The slow depolarization phase is mediated by activation of "funny current" (I_{f}) and T-type Ca²⁺ channels and deactivation of slow and rapid potassium (I_{Ks} and I_{Kr}, respectively). The rate of pacemaker discharge in the SA node in a normally functioning heart is approximately in the range of about 60 to 100 beats per minute.

As mentioned in the Background section above, one treatment option for cardiac dysfunction associated with atrial fibrillation is to ablate the AV node so that conduction of atrial impulses that give rise to irregular ventricular rhythms is prevented. Since an ablated AV node is incapable of conducting SA nodal impulses to the ventricles, other structures of the heart with intrinsic pacemaking activity may attempt to take over and pace the ventricles. However, the heart rate generated by these substitute intrinsic pacemakers is not sufficient to support normal systemic circulation and hence a pacemaker with appropriate firing rate must be constituted. A method of use of the present invention includes genetically ablating the cells of the AV node so that they are incapable of transmitting rapid atrial activity during atrial fibrillation to the ventricles and a concurrent modification of other myocardial cells, such as ventricular cells or Purkinje fiber cells, into SA node-like pacemaker cells.

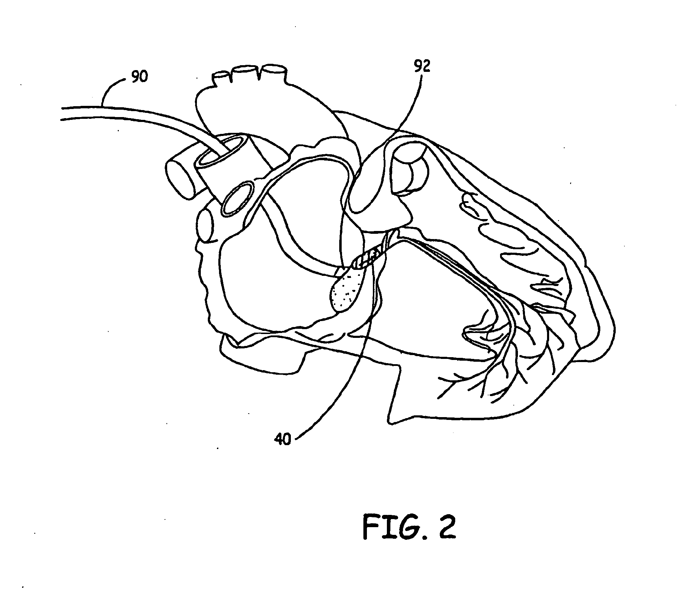

Figure 2 is a schematic diagram of the right side of a heart similar to that shown in Figure 1, wherein a guide catheter 90 is positioned for delivery of the genetic construct of the invention. A venous access site (not shown) for catheter 90 may be in a cephalic or subclavian vein and means used for venous access are well known in the art, including the Seldinger technique performed with a standard percutaneous introducer kit. Guide catheter 90 includes a lumen (not shown) extending from a proximal end (not shown) to a distal end 92 that slideably receives delivery system 80. Guide catheter 90 may have an outer diameter between approximately 0.115 inches (2.921 mm) and 0.170 inches (4,318 mm) and is of a construction well known in the art. Distal end 92 of guide catheter 80 may include an electrode (not shown) for mapping electrical activity in order to direct distal end 92 to an implant site near bundle of His 40. Alternatively, a separate mapping catheter may be used within lumen of guide catheter 90 to direct distal end 92 to an implant site near bundle of His 40, a method well known in the art.

In one embodiment of the invention, a sufficient amount of a bio-ablation composition is delivered to the cells of the AV node, where the composition modifies one or more electrophysiological properties to decrease conduction of impulses therethrough. In one embodiment, the composition includes a genetic construct or vector that decreases the excitability of the cells of the AV node, for example, by decreasing Ca²⁺ current or changing the channel activity of an ion channel. In another embodiment, in addition to delivering of the bio-ablation composition, a sufficient amount of a bio-pacemaker composition that includes a genetic construct or vector is delivered to myocardial cells, where the genetic construct modifies one or more properties of the cells to increase their intrinsic pacemaking rate. For example, a bio-pacemaker composition of the invention is delivered to cells of the Purkinje fibers or ventricular cells that will do one or more of the following: 1) increase the inward T-type Ca²⁺ current, 2) decrease Na⁺ current; 3) increase the outward K⁺ current (Iₖᵣ and Iₖₛ), or 4) increase the funny current. Myocardial cells may be modified to maximize the transformation of these cells into the primary pacemaker and to increase their intrinsic pacing rate to a level resembling that of the SA node. In one embodiment, the intrinsic pacing rate of the modified cells is increased to a level substantially identical to that of the SA node. As used herein, "resembling" or "resembles" means that the pacing rate of the modified cells is increased to a level of at least about 85% of the pacing rate of the SA node cells for a particular patient when the heart is functioning normally and "substantially identical" means that the pacing rate of the modified cells is increased to a level of at least about 95% of the pacing rate of the SA node cells for the patient when the SA node of the heart is functioning normally.

The terms "encodes", "encoding", "coding sequence", and similar terms as used herein, refer to a nucleic acid sequence that is transcribed (in the case of DNA) and translated (in the case of mRNA) into a polypeptide *in vitro* or *in vivo* when placed under control of appropriate regulatory sequences.

Biological ablation of the AV node may be accomplished by a number of methods. Since AV node cells lack Na⁺channels, the upstroke of their action potentials and hence conduction down the node, is primarily governed by L-type Ca²⁺ channels. Therefore, according to an embodiment of the invention, conduction down the AV node will be extinguished by the suppression of the activity of this channel through genetic modification. The channel is composed of five subunits, α₁, α₂, β, γ, and δ, that coassemble to form functional channels. The α₁ subunit contains the binding site for Ca²⁺ and is the primary pore forming subunit of the channel. Activity of the channel can be suppressed by genetic modifications that interrupt trafficking of this protein to the cell surface.

The α₁ subunit contains an endoplasmic reticulum retention signal that prevents trafficking of the subunit to the cell surface. The β subunit reverses the retention signal by binding to the α₁ subunit. The reversal effect of the β subunit is controlled by a signal transduction cascade involving heterotrimeric G-proteins, protein kinases, and calmodulin (CaM). Intracellular Ca²⁺ binds and activates CaM, which in turn binds the inactive form of the G-protein, kir/GEM. Kir/GEM contains a guanine binding site and cycles between an active GTP-bound state and inactive GDP-bound state. The association between kir/GEM and CaM localizes kir/GEM to cytoplasm where GDP is phosphorylated to the active form with concurrent disassociation of CaM. The active form of kir/GEM binds the β subunit and prevents the association of the α₁ and β subunits and therefore prevents trafficking and formation of functional L-type Ca²⁺ channels at the cell surface.

According to an embodiment, Ca²⁺ current is decreased by genetically modifying the AV node to express exogenous Kir/GEM. Exogenously expressed Kir/GEM will bind β subunits and prevent the trafficking of the functional channel to the cell surface. Decreased levels of the L-type Ca²⁺ channels will decrease the flow of Ca²⁺ into the cells, decrease the cell excitability, and prevent conduction of atrial impulses to the ventricles. L-type Ca²⁺ channel activity is under control of the parasympathetic nervous system via muscarinic 2 (M2) receptors. The conductance of L-type Ca²⁺ channels is modulated by its phosphorylation and dephosphorylation. Phosphorylation increases its conductance and dephosphorylation decreases conductance. This modulation of channel conductance is under direct autonomic control via stimulation by the vagus nerve. Acetylcholine released from this nerve binds M2 receptors, which are coupled to an inhibitory heterotrimic G-protein (Gᵢ). Binding of acetylcholine causes the dissociation of Gᵢ into two subunits, G_{iα} and G_{iβγ}. The G_{iα} subunit inhibits adenyl cyclase and after a series of phosphorylation steps, protein kinase A (PKA) activity is decreased. This results in dephosphorylation of L-type Ca²⁺ channels. Thus, stimulation of the vagus nerve causes a decrease in the impulses traveling through the AV node and a subsequent decrease in heart rate.

Bio-ablation of the AV node can be accomplished by genetically modifying cells of the AV node to overexpress Gᵢ. Excess Gᵢ will result in an increase in dephosphorylation of the L-type Ca²⁺ channel thereby decreasing its conductance. A decrease in channel conductance of a large number of channels will significantly decrease the Ca²⁺ current and reduce the excitability of AV node cells to block conduction therethrough. Conduction through the AV node can also be decreased or eliminated by directly suppressing the L-type Ca²⁺ channel. That is, expression can be suppressed at the transcriptional translational, or post translational level.

The AV node conducts pacemaking impulses from the SA node to the ventricles to facilitate cardiac contraction. Once the AV node has been modified as described above, it will no longer be capable of conducting the atrial impulses. Thus, it is necessary to complement the biological ablation with an alternative pacemaker. The alternative pacemaker may be an implantable pacemaker as is well known in the art. Alternatively, as described below, a biological pacemaker may be constituted.

Necessarily, when the AV node is bio-ablated, a biological pacemaker must be provided in cardiac structures that are below the AV node in the cardiac conduction system. Potential pacemaker sites include the bundle of His, major bundle branches, Purkinje fibers, or the ventricular muscle itself. The pacemaker may be generated in either the left or right ventricle. In one embodiment, for ease of delivery of a bio-pacemaker composition to the ventricles, the bio- pacemaker is generated in the RV apex or left ventricle epicardium. Alternatively, the pacemaker may be generated in the bundle of His, major bundle branches, or the Purkinje fibers.

A number of genetic modifications may be carried out in accordance with the present invention to generate bio-pacemakers. For example, myocardial cells are genetically modified to increase the transient inward Ca²⁺ current by delivering a bio-pacemaker composition to these cells. As a specific example, for ventricular cells, the bio-pacemaker composition includes a coding sequence that encodes a T-type Ca²⁺ channel resulting in the exogenous expression of T-type Ca²⁺ channels. Exogenous expression of this channel will facilitate the depolarization characteristics of ventricular cells necessary to increase their intrinsic pacing rate.

According to another embodiment, ventricular cells are genetically modified to increase the funny current (Iᵣ) by delivering a genetic construct including a coding sequence that encodes a channel producing the funny current. One such coding sequence is the hyperpolarization-activated cation channel gene (HCN) or a portion thereof. One or more isoforms of HCN may be used in the invention. Four isoforms of the HCN family, HCN1 , HCN2, HCN3, and HCN4 have been identified. Recent studies suggest that the HCN4 isoform is the predominant subunit encoding for the cardiac funny current channel in the SA node. (See, e.g., "Molecular Characterization of the Hyperpolarization-activated Cation Channel in Rabbit Heart Sinoatrial Node," J. Biol. Chem. 274:12835-12839 (1999)).

According to another embodiment, myocardial cells below the AV node, such as, ventricular cells, can be modified to decrease sodium current (I_{Na}). In the SA node, L-type Ca²⁺ channels mediate the upstroke of action potentials. However, in ventricular cells, the upstroke of action potentials is mediated by I_{Na} resulting in a faster upstroke compared to that of the SA node. The upstroke of ventricular cell APs can be slowed by suppression of endogenous I_{Na}. Endogenous I_{Na} expression can be suppressed by introducing polynucleotide sequences or molecules that interfere with the expression of wild type I_{Na}. According to another embodiment, ventricular cells are modified to decrease I_{Kl}. In the SA node, automacity is the product of an unstable resting potential, so that depolarization occurs immediately after each action potential. Ventricular cells, on the other hand, exhibit a stable resting potential due to a large I_{Kl}. The pacemaking rate of ventricular cells.can be increased by destabilizing the resting potential. The resting potential can be destabilized and the rate of action potentials increased by suppressing endogenous I_{Kl} . Inwardly rectifying K⁺ channels are responsible for I_{Kl} and can be suppressed as described herein below.

Any combination or all of the above-described genetic manipulations may be carried out. For example, myocardial cells located downstream of the AV node, such as, ventricular cells, may be modified to elicit exogenous expression of the T-type Ca²⁺ channel. Alternatively, the cells may be modified so that they express both T-type Ca²⁺ channels and I_{f} channels. In one embodiment, the cells are modified so as to express one or more of the above-mentioned channels concurrently with suppression of one or more of endogenous Na⁺ channels or I_{Kl}.

Genetic modifications of other cardiac cells to generate a bio-pacemaker in accordance with the present invention may also be carried out. For example, in one embodiment, the cells of the Purkinje fibers are genetically modified to increase the inward transient Ca²⁺ current by delivering a bio-pacemaker composition to these cells. As a specific example, the composition includes a coding sequence that encodes a T-type Ca²⁺ channel resulting in the exogenous expression of T-type Ca²⁺ channels. Exogenous expression of this channel will facilitate the depolarization characteristics of Purkinje fiber cells necessary to increase their intrinsic pacing rate.

In accordance with another embodiment of the invention, genetic modifications to Purkinje fiber cells to increase I_{K} are undertaken. Relative to the SA node, Purkinje fiber cells express low levels of the channels, Iₖᵣ and Iₖₛ that are responsible for I_{K}. The Iₖᵣ channel is comprised of two subunits (a and β), encoded by erg1 and MiRP, respectively, that coassemble to produce functional channels. Heterologous expression of erg1 produces potassium currents similar to Iₖᵣ. The I_{Ks} channel is comprised of an a subunit encoded by KvLQT1 and an accessory β subunit encoded by minK. Coassembly of both these subunits is necessary to elicit Iₖₛ.

In the SA node, deactivation of Iₖᵣ and I_{Ks} during late phase repolarization facilitates depolarization at a rate sufficient to maintain an adequate heart rate. Weak I_{K} in the cells of the Purkinje fibers increases action potential duration (APD). Extended APD implies that the intrinsic pacemaking rate of the Purkinje cells is comparatively slow and insufficient to sustain normal circulation. The APD of Purkinje fiber cells can be shortened to more closely resemble the APD of the SA node by increasing I_{K} with exogenous expression of I_{Kr} and/or I_{KS}. Delivering a bio-pacemaker composition including coding sequences for I_{Kr} or I_{Ks} to Purkinje fiber cell can increase exogenous expression of these channels. For exogenous expression of I_{Kr}, the composition may include the ergl coding sequence, or alternatively, both ergl and MiRP coding sequences may be delivered to the cells. For exogenous expression of I_{Ks}, the bio-pacemaker composition includes the minK and KvLQT1 coding sequences.

According to another embodiment, Purkinje fiber cells, can be modified to decrease sodium current (I_{Na}). In the SA node, L-type Ca²⁺ channels mediate the upstroke of action potentials. However, the upstroke of action potentials in wild type Purkinje fiber cells is mediated by I_{Na}. Mediation by I_{Na} results in a more rapid upstroke compared to that of the SA node. The upstroke of Purkinje fiber AP's can be slowed by suppression of endogenous I_{Na}. Endogenous I_{Na} expression can be suppressed by introducing polynucleotide sequences or molecules that interfere with the expression of wild type of I_{Na}.

The pacing rate of any cardiac cell type is the product of the composition of channels expressed by the cell as well as electrotonic influences exerted by neighboring cells. For example, evidence suggests that the ventricles exert electrotonic influences on the Purkinje cells at the Purkinje-ventricular junction, thereby inhibiting its pacing rate. Thus, to be effective, proposed genetic modifications must take into account the wild type channel expression as well as influences exerted by neighboring cells.

The electrotonic influences of the ventricles can be decreased by partial electrical uncoupling of Purkinje fibers from the neighboring ventricular cells. Since electrical impulse spread through the ventricles via gap junctions, uncoupling the gap junctions in the vicinity of the genetic modification can help to decrease the electronic load on the Purkinje cell derived bio-pacemaker and enhance its pacing rate. As is clear, this modification is particularly useful where the genetic modifications are performed in the more distal portions of the Purkinje fibers that are embedded in the ventricular endocardium.

Gap junctions can be uncoupled by interfering with the formation of connexons. Ventricular gap junctions can be preferentially uncoupled while leaving the gap junctions of the Purkinje cells or other cells intact, by the targeted interference of connexin 43 (CX43), the predominant form of connexin protein in the ventricular gap junctions. Accordingly, the present invention provides for the preferential uncoupling of ventricular gap junctions in the area of the Purkinje-ventricular junction, and other alternate embodiments, in the immediate area of any of the preceding genetic modifications.

Any combination or all of the above-described genetic manipulations may be carried out. For example, the cells of the conduction system, (e.g., Purkinje fibers) may be modified as to elicit exogenous expression of the T-type Ca²⁺ channel. Alternatively, the cells of the conduction system are modified so that they express the T-type Ca²⁺ channel, I_{Kr} and I_{Ks}. In the SA node, all three channels contribute to the pacemaking rate, for example, an alternative embodiment is to modify all three characteristics of the Purkinje fibers simultaneously or sequentially. Alternatively, the cells may be modified so as to express one or more of the above-mentioned channels concurrently with suppression of endogenous I_{Na}+ (i.e. Na⁺ current).

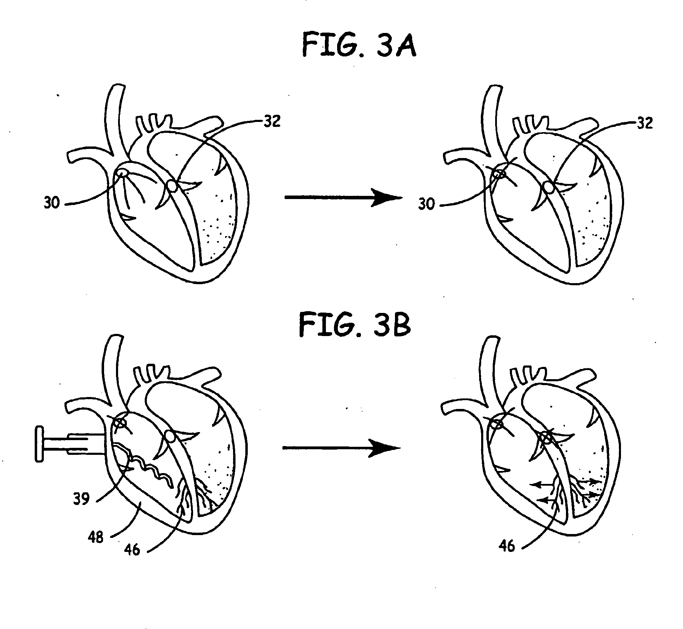

The schematics of Figures 3A and 3B illustrate the effect of the genetic modification of the invention. Figure 3A illustrates a heart with the AV node bio-ablated according to a method of the invention. After delivery of the bio-ablation composition to the AV node and subsequent modification, impulse generated by the SA node 30 is no longer conducted through the AV node 32. Figure 3B illustrates the delivery of a bio-pacemaker composition comprising genetic vector or construct 39 to the Purkinje fiber cells 46 or ventricular cells 48. After the genetic vector or construct has been delivered to the host cell and modified gene expression has occurred, at least in a portion of the cells, their electrophysiology will be altered to more closely resemble that of a normally functioning SA node.

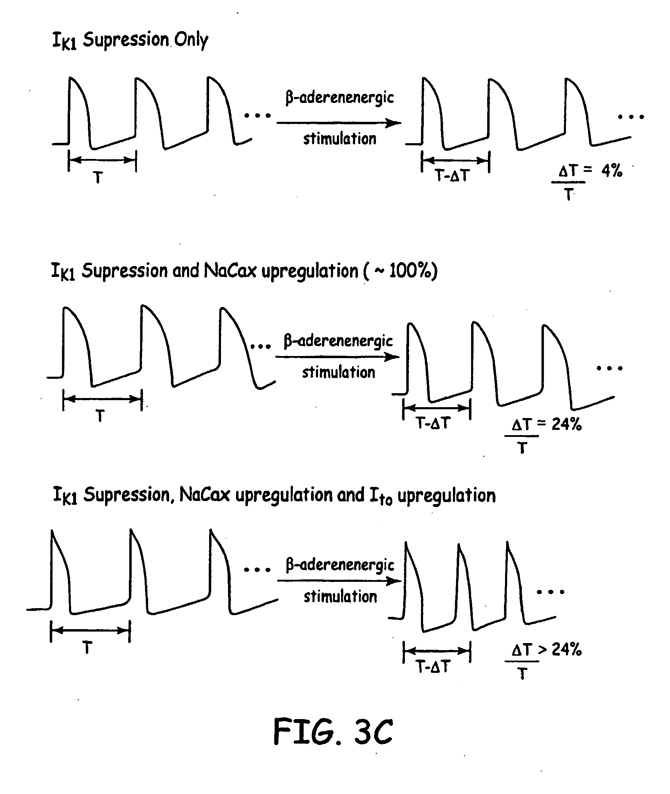

A biological pacemaker should have an adequate autonomic response so that its pacing rate adapts to changes in metabolic demand of the body. Consequently, the cardiac output should scale appropriately with the physical activity. For a bio-pacemaker obtained by predominantly suppressing I_{Kl}, alternate channel modifications are necessary to obtain adequate rate response. The pacemaking mechanism of a biological pacemaker obtained using I_{Kl} suppression involves an imbalance between inward leakage current (carried primarily via Ca²⁺ and Na⁺ ions) and outward I_{Kl} current in conditions of downregulated I_{Kl}. The conditions lead to Ca²⁺ overload in the cell, and an increase in forward mode of Na-Ca²⁺ exchanger NaCaX), another sarcolemmal ion channel, resulting in net inward depolarizing current (Silva and Rudy, Circ Res, 2003; 92:261-263). β-aderenergic stimulation causes several changes in the ionic processes of the cell (see table on page 262 in Silva and Rudy, Circ Res, 2003; 92:261-263). However, the net effect of β-aderenergic stimulation is a mere 4% increase in the heart rate. To change the heart rate by an appreciable amount (>20%), NaCaX needs to be upregulated. Thus, in another embodiment of the invention, a viral vector composition is delivered that encodes for the NaCaX and upregulates this ion channel by ∼100%. Figure 3C illustrates the effect of upregulating NaCaX on β-aderenergic response of bio-pacemaker cells which, inter alia, results in higher cycles of cardiac activity.

The second factor that prevents the heart rate from responding adequately to β-aderenergic stimulation is an increase in action potential duration in view of an increasing L-type Ca²⁺ current with sympathetic stimulation. Thus, any modification that decreases the action potential duration would aid in achieving the right rate response. One such modification is an upregulation of transient K⁺ outward current Iₜₒ, which is responsible for early repolarization phase in the action potential of cardiac cells (this is more pronounced in the epicardial cells). Thus, as another embodiment a viral vector composition is delivered to upregulate the Ito at the site of the biological pacemaker. Figure 3C also illustrates the effect of upregulating Iₜₒ concomitant with upregulation of NaCaX on β-aderenergic response of bio-pacemaker cells.

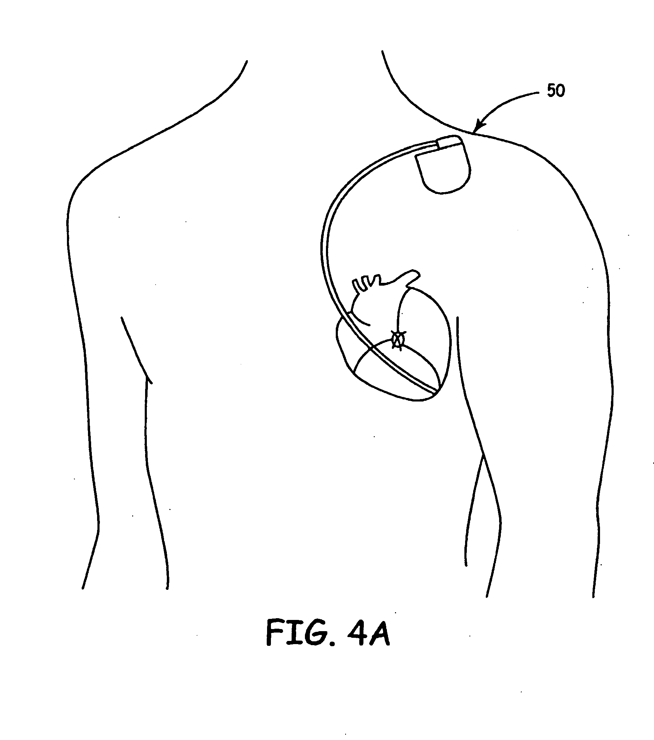

Cardiac disease often onsets suddenly, and the patient may require immediate pacemaker treatment. As is well known, the effects of gene or polynucleotide transfer may not be appreciated for as long as several days. Thus, as depicted in Figure 4A, an implantable pacemaker 50 is implemented with the bio-pacemaker of the invention to act as a bridge in the days following the genetic treatment of the present invention before full expression or suppression of channels or other proteins is accomplished. In this embodiment, an implantable pacemaker 50 is implanted consistent with methods well known in the art. The implantable pacemaker 50 may be adapted or programmed to serve several purposes. For example, the implantable pacemaker 50 may act as backup to the bio-pacemaker of the present invention. In the event the bio-pacemaker fails, malfunctions, or a slowing of the pacing rate is sensed, the implantable pacemaker 50 may be activated to take over the pacing function. Further, the implantable pacemaker may supplement the activity of the bio-pacemaker in the event the bio-pacemaker fails to produce sufficient stimulation. Other purposes for employing an implantable pacemaker to supplement or to be.used with the genetic modification of the Purkinje fibers or ventricular cells will be evident to a person of ordinary skill in the art.

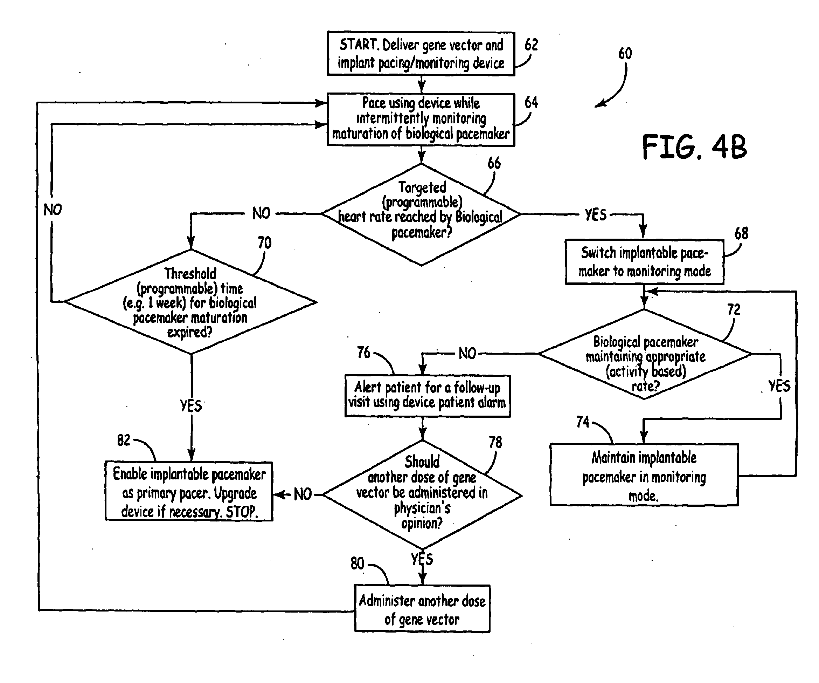

The cooperative operation of implantable pacemaker 50 and bio-pacemaker is described in Figure 4B. Specifically, one aspect of the operational logic between the implantable pacemaker 50 and the bio-pacemaker is shown. Computer implemented software logic system 60 includes logic step 62 where a gene vector is delivered to a targeted region of the cardiac conduction system and a pacemaker is implanted. Under logic step 64, the pacemaker is used to pace the patient's heart while intermittently monitoring the maturation of the biological pacemaker or the number of therapy occasions at which the gene vector that has been delivered. Under decision step 66, when the biological pacemaker reaches a targeted or programmable heart rate, the implantable medical device is switched to a monitoring mode under logic step 68. However, if the biological pacemaker has not reached the targeted heart rate, then under decision logic step 70, the time of the biological pacemaker maturation is checked to see if it has expired. If the time has expired, then the logic proceeds to enable implantable pacemaker as a primary pacemaker under logic step 82. If, on the other hand, the threshold time for the biological pacemaker has not expired, the system reverts back to logic step 64 where the device while intermittently monitoring maturation of the biological pacemaker does pacing. Referring now to logic step 66, if the targeted heart rate is reached by the biological pacemaker, then under logic step 68, the implantable pacemaker is switched to only monitor the operation of the biological pacemaker. Subsequently, under logic step 72, the biological pacemaker is checked to see whether it is maintaining the appropriate rate. If the appropriate pacing rate is maintained by the biological pacemaker, the implantable pacemaker is maintained in a monitoring mode and in the alternative, if the biological pacemaker is not keeping the appropriate rate, a patient alert is triggered to make the patient aware for a follow-up visit. Typically, the alert is communicated via device patient alarm, or other equivalent perceptible means. Further, under logic step 78, the system looks to see whether another dose of gene vector should be administered based upon a physician's opinion. If such a dose is confirmed, another dose of gene vector under logic step 80 is administered and the logic reverts back to logic step 64 to pace using the device while intermittently monitoring the maturation of the biological pacemaker. In the alternate, if the administration of another dose of gene vector is not advisable, the system reverts to logic step 82 where it would enable the implantable pacemaker to operate as the primary pacer. Further, the implantable pacemaker may act as backup to the bio-pacemaker of the present invention. In the event the bio-pacemaker fails, malfunctions, or a slowing in the pacing rate is sensed, the implantable pacemaker may be activated to take over the pacing function. Specifically, the implantable pacemaker may supplement the activity of the bio-pacemaker in the event the bio-pacemaker fails to produce sufficient stimulation. Other purposes for employing an implantable pacemaker to supplement or to be used with the genetic modification of the AV node includes chronic data management for diagnostic purposes and tracking and monitoring long term performance of the genetically obtained bio-pacemaker.

Delivery of the bio-pacemaker composition or bio-ablation composition comprising a genetic construct can be carried out according to any method known in the art. For generating bio-pacemaker, it is only necessary that the genetic construct reach a small portion of the cells in an area of liminal dimension (i.e., ∼0.5-1.0 mm across) that are targeted for gene manipulation (e.g. cells of Purkinje fibers, or ventricles). The genetic construct may be injected directly into the myocardium as described by R.J. Guzman et al., Circ. Res., 73:1202-1207 (1993). The delivery step may further include increasing microvascular permeability using routine procedures, including delivering at least one permeability agent prior to or during delivery of the genetic construct. Perfusion protocols useful with the methods of the invention are generally sufficient to deliver the genetic construct to at least about 10% of cardiac myocytes in the mammal. Infusion volumes from about 0.5 to about 500 ml are useful. Methods for targeting non-viral vector genetic constructs to solid organs, for example, the heart, have been developed such as those described in U.S. Pat No. 6,376,471.

Therapeutic methods of the invention comprise delivery of an effective amount of a genetic construct of the invention to the myocardial cells, such as cardiac Purkinje fiber cells or ventricular cells, to increase the intrinsic pacing rate of these cells to resemble the pacing rate of the SA node cells. The delivery or administration may be accomplished by injection, catheter and other delivering means known in the art. A delivery system for delivering genetic material in a targeted area of the heart is described in PCT Publication No. WO 98/02150, assigned to the assignee of the present application.

The genetic construct can be delivered into a cell by, for example, transfection or transduction procedures. Transfection and transduction refer to the acquisition by a cell of new genetic material by incorporation of added nucleic acid molecules. Transfection can occur by physical or chemical methods. Many transfection techniques are known to those of ordinary skill in the art including, without limitation, calcium phosphate DNA coprecipitation, DEAE-dextrin DNA transfection, electroporation, naked plasmid adsorption, and cationic liposome-mediated transfection. Transduction refers to the process of transferring nucleic acid into a cell using a DNA or RNA virus. Suitable viral vectors for use as transducing agents include, but are not limited to, retroviral vectors, adeno associated viral vectors, vaccinia viruses, an Semliki Foret virus vectors.

### Tests are known

to determine the cardiac action potential characteristics, such as action potential duration (APD). An example of such a method related to performing such tests is disclosed by Josephson ME, Clinical Cardiac Electrophysiology: Techniques and Interpretations. Lea & Febiger. (1993), pp 22-70.

Briefly, a standard electrophysiological assay includes the following steps: providing a mammalian heart (*in vivo or ex vivo*), delivering to the heart a genetic construct or modified cells of the invention, transferring the genetic construct and/or modified cells into the heart under conditions which can allow expression of an encoded amino acid sequence; and detecting increase of at least one electrical property in the cells of the heart to which the genetic construct and/or modified cells were delivered, wherein at least one property is the pacing rate of the cells, relative to a baseline value. Baseline values will vary with respect to the particular myocardial cells chosen. Additionally, modulation of cardiac electrical properties obtained with the methods of use of the invention may be observed by performing a conventional electrocardiogram (ECG) before and after administration of the genetic construct of the invention and inspecting the ECG results. ECG patterns from a heart's electrical excitation have been well studied. Various methods are known for analyzing ECG records to measure changes in the electrical potential in the heart associated with the spread of depolarization and repolarization through the heart muscle.

In the invention, a bio-pacemaker composition and/or bio-ablation composition that includes a polynucleotide capable of increasing the expression of a particular ion channel or suppressing, in whole or in part, the expression or function of an ion channel may be made. Polynucleotides encoding the ion channel or protein of choice can be made by traditional PCR-based amplification and known cloning techniques. Alternatively, a polynucleotide of the invention can be made by automated procedures that are well known in the art. A polynucleotide of the invention should include a start codon to initiate transcription and a stop codon to terminate translation.

Suitable polynucleotides for use with the invention can be obtained from a variety of public sources including, without limitation, GenBank (National Center for Biotechnology Information (NCBI)), EMBL data library, SWISS-PROT (University of Geneva, Switzerland), the PIR-International database; and the American Type Culture Collection (ATCC)(10801 University Boulevard, Manassas, VA 20110-2209). See generally, Benson, D.A. et al, Nucl. Acids. Res., 25:1 (1997) for a description of GenBank. The particular polynucleotides useful with the present invention are readily obtained by accessing public information from GenBank.

Any DNA vector or delivery vehicle can be utilized to transfer the desired nucleotide sequence to the cells of the cardiac conduction system. For example, the transgene or transgenes may be cloned into a viral vector such as an adenoviral associated vector (AAV). Alternatively, other viral vectors such as, herpes vectors, and retroviral vectors such as lentiviral vectors may be employed. The type of viral vector selected is dependent on the target tissue and the length of the sequence to be delivered. Alternatively, non-viral delivery systems may be utilized. For example, liposome: DNA complexes, plasmid: liposome complexes, naked DNA, DNA-coated particles, or polymer based systems may be used to deliver the desired sequence to the cells. The above-mentioned delivery systems and protocols therefore can be found in Gene Targeting Protocols, Kmeic 2ed., pages 1-35 (2002) and Gene Transfer and Expression Protocols, Vol. 7, Murray ed., Pages 81-89 (1991).

AAV vectors can be constructed using techniques well known in the art. Typically, the vector is constructed so as to provide operatively linked components of control elements. For example, a typical vector includes a transcriptional initiation region, a nucleotide sequence of the protein to be expressed, and a transcriptional termination region. Typically, such an operatively linked construct will be flanked at its 5' and 3' regions with AAV ITR sequences, which are required viral cis elements. The control sequences can often be provided from promoters derived from viruses such as, polyoma, Adenovirus 2, cytomegalovirus, and Simian Virus 40. Viral regulatory sequences can be chosen to achieve a high level of expression in a variety of cells. Alternatively, ubiquitously expressing promoters, such as the early cytomegalovirus promoter can be utilized to accomplish expression in any cell type. A third alternative is the use of promoters that drive tissue specific expression. This approach is particularly useful where expression of the desired protein in non-target tissue may have deleterious effects. Thus, according to another preferred embodiment, the vector contains the proximal human brain natriuretic brain (hBNP) promoter that functions as a cardiac-specific promoter. For details on construction of such a vector see LaPointe et al., "Left Ventricular Targeting of Reporter Gene Expression In Vivo by Human BNP Promoter in an Adenoviral Vector," Am. J. Physiol. Heart Circ. Physiol., 283:H1439-45 (2002).

Vectors may also contain cardiac enhancers to increase the expression of the transgene in the targeted myocardial cells. Such enhancer elements may include the cardiac specific enhancer elements derived from Csx/Nkx2.5 regulatory regions disclosed in the published U.S. Patent Application 20020022259.

Introducing the AAV vector into a suitable host, such as yeast, bacteria, or mammalian cells, using methods well known in the art, can produce AAV viral particles carrying the sequence of choice.

A number of different constructs can be generated in accordance with the invention. For example, a construct can be produced that includes the coding sequence of the α_{lH} subunit of the T-type Ca²⁺ channel, the α and/or β subunit of I_{Kn} or the a and/or β subunits of I_{Ks}. Constructs containing the coding sequence for one or more of the channels or subunit thereof are referred to as single gene constructs. These constructs are useful when practicing embodiments that call for the introduction of only one channel sequence or when it is desired to titrate the expression of the transgenes relative to each other. Such differential expression of the channels can be accomplished by generating vectors with varied promoters or administration of differing dosages. Alternatively, multiple transgenes can be co-delivered by compound vectors as is known by those skilled in the art.

Targeted gene suppression can be accomplished by a number of techniques. In general, polynucleotides that interfere with expression of I_{Na}, I_{Kl}, I_{CaL}, or CX43 at the transcriptional, translational, or trafficking level may be administered to cells of the Purkinje fibers, AV node, or ventricles. For example, a polynucleotide that either encodes for a dominant negative form of the protein to be suppressed, functions as a decoy, or sterically blocks transcription by triplex formation may be employed. Alternatively, antisense approaches may be utilized.

Dominant negative gene suppression is achieved by introducing mutations into the wild type gene and expressing the mutated gene in a cell expressing wild type protein. The dominant negative protein acts to decrease levels of a particular protein by interfering with the assembly or function of the wild type protein. The mutations in the wild type gene may be introduced by site-directed mutagenesis.

Effective dominant negative mutations may include those directed to residues that are important for the trafficking of the protein to the cell surface or folding of the wild type protein and thereby decrease the number of functional protein at the cell surface or at the gap junction. Additional dominant negative mutations include the introduction of hydrophilic amino acids in hydrophobic transmembrane regions. Such alterations prevent the effective assembly of the channel into the cell membrane. Preferably, the dominant negative is specific to targeted gene so that the function of other proteins is not altered. Furthermore, in the case of ion channels, mutations should be designed so as to not alter ionic specificity of the channel.

A construct useful for the dominant negative suppression of I_{Na} is SCN5A R1432G. The replacement of arginine with glycine at position 1432 interferes with trafficking of the channel to the plasma membrane. Baroudi et al., "Novel Mechanism for Brugada Syndrome," Circ Res., 88:e78-e83 (2001). Decreased levels of the channel at the cell surface will decrease I_{Na}. A construct useful for the dominant negative suppression of I_{Kl} is Kir2.1 GYG11-146AAA. Miake et al. "Biological Pacemaker Created by Gene Transfer," Nature 419:132-133 (2002). L-type Ca²⁺ channel expression may be effectively suppressed the use of the dominant negative Ca(v)1.2 with an ascidian 3-domain type alpha 1 subunit. For a description of this dominant negative see Ebihara et al. Co-expression of Ca(v) 1.2 Protein Lacking an N-terminus and the First Domain Specifically Suppresses L-type Calcium Channel Activity," FEBS 529:203-207 (2002). Suppression of I_{Na}, I_{Kl}, I_{CaL}, and/or CX43 can also be accomplished by the administration of oligonucleotides that act as a decoy for transcription factors for the relevant gene. ' Decoys function to suppress the expression of a gene by competing with native regulatory sequences. The oligonucleotide should be specific for transcription factors that regulate the relevant genes. The oligonucleotide may be administered to the cells of the Purkinje fibers or associated structures (e.g., bundle of HIS), AV node, or ventricles by techniques well known in the art.

The invention may also be practiced employing triple helix technology to suppress expression of target genes. Thus, a single strand oligonucleotide may be introduced to the cells of the targeted myocardial cells (e.g. Purkinje fibers, AV node, or ventricles). Suppression of a targeted gene is accomplished by inhibition of transcription via the formation of a triple helix structure comprised of the targeted double strand DNA sequence and the oligonucleotide. Potential triple helix sites may be identified using computer software to search targeted gene sequence with a minimum of 80% purine over a 15 basepair stretch. The oligonucleotide may be synthesized with 3' propanolamine to protect against 3' exonucleases present in cells. For a discussion of triple helix techniques see Vasquez et al. Triplex-directed site-specific genome modification. Gene Targeting Protocols, Kmiec 2ed., pages 183-200 (2000).

In accordance with the invention, expression of any particular protein may also be suppressed using antisense techniques. Antisense therapeutics is based on the ability of an antisense sequence to bind to mRNA and block translation. Antisense oligonucleotides must have high specificity for the target gene to avoid disruption of non-targeted gene expression. Artificial antisense oligodeoxyribonucleotides are favored because they can be synthesized easily, are readily transferred to the cytoplasm of myocardial cells using liposomes, and resist nuclease activity.

Constructs of the present invention can be targeted to cells of the Purkinje network by methods known to those skilled in the art. Advantage can be taken of the expression of cell surface receptors unique to specific cells. For example, one such receptor, preferentially expressed on the surface of Purkinje cells, is the cysteinyl leukotriene 2 receptor (CysLT₂). This receptor distinguishes Purkinje cells from neighboring cells such as ventricular cells and can be utilized to target constructs of the invention preferentially to Purkinje cells. However, it is to be understood that in the practice of the present invention, any receptor specific to Purkinje cells may be utilized for specific targeting. Targeted delivery requires the modification of vehicle delivering the construct. Several methods for modification of viral vectors are possible. For example, viral protein capsids or proteins of the viral envelope may be biotinylated for subsequent coupling to a biotinylated antibody directed against a specific receptor or ligand therefore via a streptavidin bridge.

Alternatively, the viral delivery vehicle may be genetically modified so that it expresses a protein ligand for a specific receptor. The gene for the ligand is introduced within the coding sequence of a viral surface protein by for example, insertional mutagenesis, such that a fusion protein including the ligand is expressed on the surface of the virus. For details on this technique see Han et al., "Ligand-Directed Retroviral Targeting of Human Breast Cancer Cells," Proc. Natl. Acad Sci., 92:9747-9751 (1995). Viral delivery vehicles may also be genetically modified to express fusion proteins displaying, at a minimum, the antigen-binding site of an antibody directed against the target receptor. See e.g., Jiang et al., "Cell-Type-Specific Gene Transfer into Human Cells with Retroviral Vectors That Display Single-Chain Antibodies," J. Virol., 72: 10148-10156 (1998).

Construct delivery vehicles may also be targeted to specific cells types utilizing bispecific antibodies produced by the fusion of anti-viral antibody with anti-target cell antibody. For details on this technique see Haisma et al., "Targeting of Adenoviral Vectors Through a Bispecific Single-Chain Antibody," Cancer Gene Ther., 7:901-904 (2000) and Watkins et al., "The 'Adenobody' Approach to Viral Targeting: Specific and Enhanced Adenoviral Gene Delivery," Gene Ther., 4:1004-1012 (1997).

Targeted construct delivery provides numerous advantages including increased transduction efficiency and the avoidance of genetic modification of cells in which the modification would have deleterious effects on the patient. Any technique for targeted gene therapy may be employed to target the construct of the invention to cells of interest.

As will be appreciated by those skilled in the art, the genetic manipulations described here may be practiced on stem cells without departing from the scope of the invention. The genetically modified stem cells can then be administered to the desired myocardial cells to elicit pacemaking activity or suppress conduction characteristics. For example, cardiac myocardial cells derived from stem cells may be treated with the genetic procedures described herein and implanted into a heart (e.g. ventricular muscle) with a catheter or by direct epicardial injection into the ventricular tissue.

The invention will be further described with reference to the following non-limiting examples. It will be apparent to those skilled in the art that many changes can be made in the embodiments described in the examples without departing from the scope of the present invention. Thus, the scope of the present invention should not be limited to the embodiments described in this application, but only by the embodiments described by the language of the claims and the equivalents of those embodiments.

### EXAMPLE 1: Genetic Ablation and Increased Intrinsic Pacemaking Rate of Genetically Modified Purkinje Fibers:

### Construction of rAAV Cloning Plasmids Construct Generation

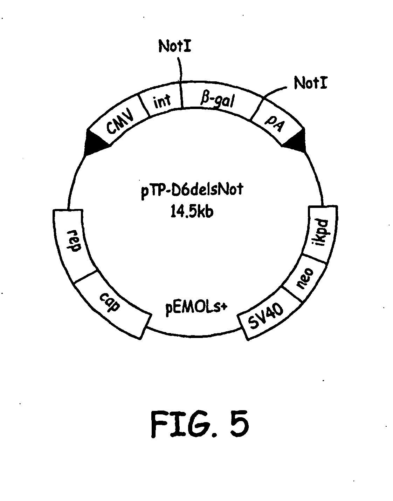

Genetic constructs useful in the bio-pacemaker composition and bio-ablation composition of the invention can be generated using traditional techniques as described Schnepp and Clark in Gene Therapy Protocol, Morgan 2ed., pages 490-510 (2002). Certain constructs of the present invention are generated by cloning the cDNA of minK (sequence listed in U.S. Pat. No. 6,323,026), KvLQT1 ((GenBank accession No. AJ006345), erg (GenBank Accession No. AB009057), NaCaX (GenBank Accession No. S69069), hₜₒ (GenBank Accession No. AF137071), or α1H (GenBank Accession No. AF051946) into the rAAV producer plasmid, pTP-D6deltaNot. Other constructs are generated by cloning cDNA of Kir/GEM (GenBank accession No. U13052) or Gᵢ (GenBank accession no. AH001470) into the producer plasmid. Yet other constructs are produced by cloning in cDNA of T-type Ca²⁺ channel (GenBank accession No. AF051946) or I_{f} (GenbBank accession No. NM005477) into the producer plasmid. The pTP-D6deltanot producer plasmid is shown in Fig. 5 and includes AAV rep and cap genes, a neomycin resistance gene flanked by the SV40 promoter and thymidine kinase polyadenylation signal, and a gene expression cassette flanked by AAV inverted terminal repeats (ITRs) and comprising the CMV promoter, SV40 large T-antigen intron, and polyadenylation signal, and beta galactocidase gene flanked by two unique NotI restriction sites. The cDNA for the channel of choice replaces the beta galactocidase gene by excising the gene using NotI restriction enzymes and cloning in the cDNA for the transgene. The resulting producer plasmid is used to produce rAAV particles. Alternatively, other constructs can be generated substituting alternative promoters (e.g., a rAAV producer plasmid containing alternate promoters).

The producer plasmid containing the transgene sequence is amplified by transformation of DH5-alpha *E*. *coli* and producing colonies screened by neomycin resistance. Producer plasmid is then isolated from resistant colonies and co-transfected with wild type adenovirus 5 (E1 deleted) into suitable host cells Such as HeLA (for a discussion of the use of HeLA cells to produce rAAV particles see Clark et al., "Cell Lines for the Production of Recombinant Adeno-Associated Virus," Human. Gene Ther. 6:1329-1341 (1995). Host cells containing the vector are purified using ammonium sulfate followed by double cesium banding. The bands containing the viral particle are isolated from the cesium chloride preparation and dialyzed into a Tris buffer, or other suitable buffer.

### Generation of Dominant Negative Constructs

Dominant negative constructs are generated by synthesizing oligonucleotides comprising the gene coding sequence, including the dominant negative mutation using the site-directed mutagenesis system available in the Altered Sites® II Systems (Promega, Madison WI). This oligonucleotide is used as a primer to produce a plasmid containing the hybrid gene sequence. *E. coli* are transformed with the hybrid plasmid for amplification of the mutagenic gene. The mutant sequence is excised from the hybrid plasmid and cloned into the cloning plasmid as described above. CX43 is suppressed using the dominant negative A253V. The dominant negative sequence is produced by synthesizing a synthetic oligonucleotide including the A253V substitution as described above. The wild type sequence is deposited at GenBank accession No. AF 151980.

Suppression of endogenous I_{Na} is accomplished by delivery of a construct including the coding sequence of SCN5A including the R1432G mutation. The wild type sequence for SCNA5 is deposited at GenBank accession No. NM00035. The dominant negative sequence and construct is generated as described above.

Suppression of endogenous I_{Kl} is accomplished by delivery of a construct including the coding sequence of Kir2.1 GYG 144-146AAA. The wild type sequence for Kir2.1 is deposited at GenBank accession no. AF153820.

### In vivo Vector Administration

Adult guinea pigs are infected by intramuscular injection (via catheter) of a solution of saline with a viral concentration range of approximately 3X10¹⁰ to 3X10¹⁴ plaque forming units (PFU). For targeted injection to the right side of the heart, the catheter is guided to the right atrium, either via the superior vena cava or inferior vena cava, which by itself is accessed via one of the femoral veins. The right ventricle is then accessed by guiding the catheter through the tricuspid valve to target the left side of the heart, left atrium is accessed from the right atrium via the septum primum. From the left atrium, the catheter is guided through the bicuspid valve to the left ventricle. The AV node is targeted by direct perfusion into the AV nodal branch of the right coronary artery. The left ventricle epicardium is targeted via the coronary sinus.

## Claims

1. A kit comprising a bio-ablation composition and one or both of an implantable pacemaker and a bio-pacemaker composition comprising a coding sequence that encodes and expresses a molecule that increases the pacing rate of myocardial cells; wherein the bio-ablation composition comprises either:
i) a first coding sequence that encodes and expresses, in atrioventricular node cells, a molecule that decreases expression of L-type Ca²⁺ channels and thereby suppresses cellular excitability or
ii) a second coding sequence that encodes and expresses a protein that decreases the conductance of L-type Ca²⁺ channels responsible for cellular excitability, wherein expression of both the first and second sequences is effective to substantially extinguish conduction through the atrioventricular node; wherein the first coding sequence encodes and expresses kir/GEM in atrioventricular node cells and the second coding sequence encodes and expresses Gi in atrioventricular node cells; wherein expression of both the first and second sequences is effective to substantially extinguish conduction through the atrioventricular node;
and further wherein if the kit includes a bio-pacemaker composition, the coding sequence encodes one or more of:
Ergl, MiRP, MinK, or KvLQTl, a T-type Ca²⁺ channel or subunit thereof, a dominant negative form of a connexin, a dominant negative form of wild type sodium channels, a HCN isoform, one or more molecules that suppress I_{Kl}, a molecule that suppresses I_{Na}.

## Patentansprüche

1. Kit bzw. Satz mit einer biologischen Ablationszusammensetzung und einem implantierbaren Schrittmacher und/oder einer biologischen Schrittmacherzusammensetzung mit einer Kodiersequenz, die ein Molekül, das die Schrittfolgerate von Myokardzellen erhöht, kodiert und exprimiert, wobei die biologische Ablationszusammensetzung entweder umfasst
i) eine erste Kodiersequenz, die in atriventrikulären Knotenzellen ein Molekühl kodiert und exprimiert, das die Exprimierung von Ca²⁺-Kanälen des L-Typs verringert und hierdurch die zelluläre Erregbarkeit bzw. Reaktion auf Reize unterdrückt, oder
ii) eine zweite Kodiersequenz, die ein Protein kodiert und exprimiert, das den Leitwert bzw. die Konduktanz der Ca²⁺-Kanäle des L-Typs, verantwortlich für die zelluläre Erregbarkeit, herabsetzt, wobei die Exprimierung sowohl der ersten als auch der zweiten Sequenz wirksam ist, um die Fortführung bzw. Fortleitung durch den atrioventrikulären Knoten im Wesentlichen zu beseitigen, und wobei die erste Kodiersequenz kir/GEM in atrioventrikulären Knotenzellen kodiert und exprimiert und die zweite Kodiersequenz Gi in atrioventrikulären Knotenzellen kodiert und exprimiert, und wobei die Exprimierung sowohl der ersten als auch der zweiten Sequenz wirksam ist, um die Fortführung bzw. Fortleitung durch den atrioventrikulären Knoten im Wesentlichen zu beseitigen,
und wobei weiterhin die Kodiersequenz, wenn der Kit eine biologische Schrittmacherzusammensetzung umfasst, eines oder mehrere der nachfolgenden Elemente kodiert:
Ergl, MiRP, MinK oder KvLQTl, ein Ca²⁺-Kanal vom T-Typ oder Untereinheiten hiervon, eine dominante negative Form einer Konnexine, eine dominante negative Form von Natrium-Kanälen des wilden Typs, eine HCN Isoform, ein oder mehrere Moleküle, die I_{Kl} unterdrücken, ein Molekül, das I_{Na} unterdrückt.

## Revendications

1. Kit comportant une composition de bio-ablation et un élément parmi un stimulateur cardiaque implantable et une composition de bio-stimulateur cardiaque, ou les deux, comportant une séquence de codage qui code et exprime une molécule qui augmente la fréquence de stimulation des cellules myocardiques ; dans lequel la composition de bio-ablation comporte :
i) soit une première séquence de codage qui code et exprime, dans les cellules du noeud atrioventriculaire, une molécule qui réduit l'expression des canaux Ca²⁺ de type L et atténue ainsi l'excitabilité cellulaire
ii) soit une seconde séquence de codage qui code et exprime une protéine qui réduit la conductance des canaux Ca²⁺ de type L responsables de l'excitabilité cellulaire, dans lequel l'expression à la fois des première et seconde séquences est efficace pour mettre sensiblement fin à la conduction à travers le noeud atrioventriculaire ; dans lequel la première séquence de codage code et exprime kir/GEM dans les cellules du noeud atrioventriculaire et la seconde séquence de codage code et exprime Gi dans les cellules du noeud atrioventriculaire ; dans lequel l'expression à la fois des première et seconde séquences est efficace pour mettre sensiblement fin à la conduction à travers le noeud atrioventriculaire ;
et en outre dans lequel, si le kit inclut une composition de bio-stimulateur cardiaque, la séquence de codage code un ou plusieurs éléments parmi :
Ergl, MiRP, MinK ou KvLQT1, un canal Ca²⁺ de type T ou une sous-unité de celui-ci, une forme négative dominante d'une connexine, une forme négative dominante de canaux de sodium de type sauvage, une isoforme de HCN, une ou des molécules qui atténuent I_{K1}, une molécule qui atténue I_{Na}.
